# EUROPEAN PATENT SPECIFICATION

(11) **EP 1 422 237 B1**
(45) Date of publication and mention of the grant of the patent: **14.02.2007**
(21) Application number: 03026521.9
(22) Date of filing: 18.11.2003
(51) Int. Cl.: C07K 14/155, C07K 14/16, C12P 21/02

(54) **Methods for the recombinant production of antifusogenic peptides**
Verfahren zur rekombinanten Herstellung von Antifusogenikpeptide
Procédé de production recombinante de peptides antifusogeniques

(30) Priority: 19.11.2002 EP 02025618; 17.01.2003 EP 03000988
(43) Date of publication of application: 26.05.2004
(73) Proprietor: F. HOFFMANN-LA ROCHE AG, 4070 Basel (CH)
(72) Inventor: Kaczmarek, Alexandra, 82377 Penzberg (DE); Kopetzki, Erhard, 82377 Penzberg (DE); Schantz, Christian, 85402 Thalhausen (DE); Seeber, Stefan, 82377 Penzberg (DE)
(74) Representative: Schreiner, Siegfried

(56) References cited:
- WO-A-00/69902
- WO-A-01/44286
- WO-A-02/103026
- MISAWA SATORU ET AL: "Refolding of therapeutic proteins produced in Escherichia coli as inclusion bodies" BIOPOLYMERS, NEW YORK, NY, US, vol. 51, no. 4, 1999, pages 297-307, XP002192115 ISSN: 0006-3525
- ROOT MICHAEL J ET AL: "Protein design of an HIV-1 entry inhibitor" SCIENCE, AMERICAN ASSOCIATION FOR THE ADVANCEMENT OF SCIENCE,, US, vol. 291, no. 5505, 2 February 2001 (2001-02-02), pages 884-888, XP002170634 ISSN: 0036-8075

## Description

The invention relates to methods for the recombinant production of peptides which inhibit the fusion of viruses with membranes of target cells. In particular, this invention relates to the recombinant production of peptidic inhibitors of lentivirus such as human immunodeficiency virus (HIV), Simian immunodeficiency virus (SIV), measles virus (MEV), influenza viruses such as respiratory syncytical virus (RSV) or human parainfluenza virus (HPV).

### Background of the Invention

Fusion of viruses with cellular membranes is an essential step for the entry of enveloped viruses, such as HIV-I, HIV-II, RSV, measles virus, influenza virus, parainfluenza virus, Epstein-Barr virus and hepatitis virus, into cells. After having entered the cell the cascade of viral replication may be initiated resulting in viral infection.

HIV is a member of the lentivirus genus, which includes retroviruses that possess complex genomes and exhibit cone-shaped capsid core particles. Other examples of lentiviruses include the simian immunodeficiency virus (SIV), visna virus, and equine infectious anemia virus (EIAV). Like all retroviruses, HIV's genome is encoded by RNA, which is reverse-transcribed to viral DNA by the viral reverse transcriptase (RT) upon entering a new host cell. Influenza viruses and their cell entry mechanisms are described by Bullough, P.A., et al., Nature 371 (1994) 37-43; Carr, C.M., and Kim, P.S., Cell 73 (1993) 823-832; and Wilson, I.A., et al., Nature 289 (1981) 366-373.

All lentiviruses are enveloped by a lipid bilayer that is derived from the membrane of the host cell. Exposed surface glycoproteins (SU, gp120) are anchored to the virus via interactions with the transmembrane protein (TM, gp41). The lipid bilayer also contains several cellular membrane proteins derived from the host cell, including major histocompatibility antigens, actin and ubiquitin (Arthur, L.O., et al., Science 258 (1992) 1935-1938). A matrix shell comprising approximately 2000 copies of the matrix protein (MA, p17) lines the inner surface of the viral membrane, and a conical capsid core particle comprising ca. 2000 copies of the capsid protein (CA, p24) is located in the center of the virus. The capsid particle encapsidates two copies of the unspliced viral genome, which is stabilized as a ribonucleoprotein complex with ca. 2000 copies of the nucleocapsid protein (NC, p7), and also contains three essential virally encoded enzymes: protease (PR), reverse transcriptase (RT) and integrase (IN). Virus particles also package the accessory proteins, Nef, Vif and Vpr. Three additional accessory proteins that function in the host cell, Rev, Tat and Vpu, do not appear to be packaged.

In the case of HIV, viral entry is associated with the HIV envelope surface glycoproteins (Lawless, M.K., et al., Biochemistry 35 (1996) 13697-13708; and Turner, B.G., and Summers, M.F., J. Mol. Biol. 285 (1999) 1-32). In the case of HIV-I, this surface protein is synthesized as a single 160 kD precursor protein, which is cleaved by a cellular protease into two glycoproteins gp-41 and gp-120. gp-41 is a transmembrane protein, and gp-120 is an extracellular protein_which remains non-covalently associated with gp-41 in a trimeric or multimeric form (Hammarskjöld, M.-L., et al., Biochim. Biophys. Acta 989 (1989) 269-280). HIV is targeted to CD4⁺ lymphocytes because the CD4 surface protein acts as the cellular receptor for the HIV-I virus. Viral entry into cells is dependent upon gp-120 binding to the cellular CD4⁺ receptor molecules while gp-41 anchors the envelope glycoprotein complex in the viral membrane and mediates membrane fusion (McDougal, J.S., et al., Science 231 (1986) 382-385; and Maddon, P.J., et al., Cell 47 (1986) 333-348).

gp41 is the transmembrane subunit that mediates fusion of viral and cellular membranes. The gp41 ectodomain core is a six-helix bundle composed of three helical hairpins, each consisting of an N helix paired with an antiparallel C helix (Chan, D.C., et al., Cell 89 (1997) 263-273; Weissenhorn, W., et al., Nature 387 (1997) 426-430; Tan, K., et al., Proc. Natl. Acad. Sci. USA 94 (1997) 12303-12308). The N helices form an interior, trimeric coiled coil with three conserved, hydrophobic grooves; a C helix packs into each of these grooves. This structure likely corresponds to the core of the fusion-active state of gp41. According to Chan, D.C., et al., Proc. Natl. Acad. Sci. USA 95 (1998) 15613-15617, there is evidence that a prominent cavity in the coiled coil of the HIV type 1 gp41 is an attractive drug target.

It is assumed that the mechanism by which gp-41 mediates membrane fusion may involve the formation of a coiled-coil trimer, which is thought to drive the transition from resting to fusogenic states, as is described, for example, for influenza hemagglutinin (Wilson, I.A., et al., Nature 289 (1981) 366-373; Carr, C.M., and Kim, P.S., Cell 73 (1993) 823-832; Bullough, P.A., et al., Nature 371 (1994) 37-43).

C peptides (peptides corresponding to the C helix) of enveloped viruses, such as DP178 and C34, potently inhibit membrane fusion by both laboratory-adapted strains and primary isolates of HIV-1 (Malashkevich, V.N., et al., Proc. Natl. Acad. Sci. USA 95 (1998) 9134-9139; Wild, C.T., et al., Proc. Natl. Acad. Sci. USA 91 (1994) 9770-9774). A Phase I clinical trial with the C peptide DP 178 suggests that it has antiviral activity in vivo, resulting in reduced viral loads (Kilby, J.M., et al., Nature Medicine 4 (1998) 1302-1307). The structural features of the gp41 core suggest that these peptides act through a dominant-negative mechanism, in which C peptides bind to the central coiled coil of gp41 and lead to its inactivation (Chan, D.C., et al., Cell 93 (1998) 681-684).

Within each coiled-coil interface is a deep cavity, formed by a cluster of residues in the N helix coiled coil, that has been proposed to be an attractive target for the development of antiviral compounds. Three residues from the C helix (Trp-628, Trp-631, and Ile-635) insert into this cavity and make extensive hydrophobic contacts. Mutational analysis indicates that two of the N-helix residues (Leu-568 and Trp-571) comprising this cavity are critical for membrane fusion activity (Cao, J., et al., J. Virol. 67 (1993) 2747-2755). Therefore, compounds that bind with high affinity to this cavity and prevent normal N and C helix pairing may be effective HIV-1 inhibitors. The residues in the cavity are highly conserved among diverse HIV-1 isolates. Moreover, a C peptide containing the cavity-binding region is much less susceptible to the evolution of resistant virus than DP178, which lacks this region (Rimsky, L.T., et al., J. Virol. 72 (1998) 986-993). These observations suggest that high-affinity ligands targeting the highly conserved coiled-coil surface, particularly its cavity, will have broad activity against diverse HIV isolates and are less likely to be bypassed by drug-escape mutants.

Fusogenic structures of envelope fusion proteins was shown from influenza, Moloney murine leukemia virus, and simian immunodeficiency virus (cit. in Chan, D.C., Proc. Natl. Acad. Sci. USA 95 (1998) 15613-15617), human respiratory syncytial virus, Ebola, human T cell leukemia virus, simian parainfluenza. It indicates a close relationship between the families of orthomyxoviridae, paramyxoviridae, retroviridae, and others like filoviridae, in which viral entry into target cells is enabled by like transmembrane glycoproteins such as gp41 of HIV-1, hemagglutinin of influenza, GP2 of Ebola and others (Zhao, X., et al., Proc. Natl. Acad. Sci. USA 97 (2000) 14172-14177).

In the state of the art, methods are described for the preparation of peptidic inhibitors (C-peptides) (see, e.g., Root, M.J., et al., Science 291 (2001) 884-888; Root et al. describe peptide C37-H6 which is derived from HIV-1. HXB2 and contains residues 625-661. It was recombinantly expressed as N40-segement with a GGR-linker and a histidine tag, expressed in E.coli and purified from the soluble fraction of bacterial lysates. Zhao, X., et al. describe in Proc. Natl. Acad. Sci. USA 97 (2000) 14172-14177 a synthetic gene of recRSV-1 (human respiratory syncytial virus) which encodes Residues 153-209, a G-rich linker, residues 476-524, Factor Xa cleavage site and a his-tag. Chen, C.H., et al., describe in J. Virol. 69 (1995) 3771-3777 the recombinant expression of the extracellular domain of gp41 synthesized as fusion protein, residues 540- 686, fusion to MBP.

A number of peptidic inhibitors, also designated as antifusogenic peptides, of such membrane fusion-associated events are known, including, for example, inhibiting retroviral transmission to uninfected cells. Such peptides are described, for example, by Lambert, D.M., et al., Proc. Natl. Acad. Sci. USA 93 (1996) 2186-2191, in U.S. Patent Nos. 6,013,263; 6,017,536; and 6,020,459; and in WO 00/69902, WO 99/59615 and WO 96/40191. Further peptides inhibiting fusing associated events are described, for example, in U.S. Patent Nos. 6,093,794; 6,060,065; 6,020,459; 6,017,536; 6,013,263; 5,464,933; 5,656,480; and in WO 96/19495.

Examples of linear peptides derived from the HIV-I gp-41 ectodomain which inhibit viral fusion are DP-107 and DP-178. DP-107 is a portion of gp-41 near the N-terminal fusion peptide and has been shown to be helical, and it strongly oligomerizes in a manner consistent with coiled-coil formation (Gallaher, W.R., et al., Aids Res. Hum. Retrovirus 5 (1989) 431-440, Weissenhorn, W., et al., Nature 387 (1997) 426-430). DP-178 is derived from the C-terminal region of the gp-41 ecto-domain. (Weissenhorn, W., et al., Nature 387 (1997) 426-430). Although without discernible structure in solution this peptide and constrained analogs therefrom adopt a helical structure, bind to a groove of the N-terminal coiled-coil trimer of gp41 and thus prevent the gp41 to transform into the fusogenic state (Judice, J. K., et al., Proc. Natl. Acad. Sci. USA 94 (1997) 13426-13430).

Such short-chain peptides usually are prepared by chemical synthesis. Chemical synthesis is described, for example, by Mergler, M., et al., Tetrahedron Letters 29 (1988) 4005-4008 and 4009-4012; Andersson, L., et al., Biopolymers 55 (2000) 227-250; and by Jones, J.H., J. Pept. Sci. 6 (2000) 201-207. Further methods are described in WO 99/48513.

However, chemical peptide synthesis suffers from several drawbacks. Most important is racemization, which results in insufficient optical purity. In peptide chemistry, racemization also means epimerization at one of several chirality centers. If only 1% racemization occurs for a single coupling step, then at 100 coupling steps only 61% of the target peptide would be received (Jakubke, H.D., Peptide, Spektrum Akad. Verlag, Heidelberg (1996), p. 198). It is obvious that the number of impurities increases with growing chain length and their removal is more and more difficult and costly.

Chemical synthesis on large scale is limited by high costs and lack of availability of protected amino acid derivatives as starting materials. On the one hand, these starting materials should be used in excess to enable complete reactions, on the other hand, their use should be balanced for cost reasons, safety and environmental aspects (Andersson et al., Biopolymers 55 (2000) 227-250).

Lepage, P., et al., in Analytical Biochemistry 213 (1993) 40-48, describe recombinant methods for the production of HIV-1Rev peptides. The peptides are expressed as fusion proteins with the synthetic immunoglobulin type G (IgG) binding domains of Staphylococcus aureus protein A. The peptides have a length of about 20 amino acids, whereas the IgG-binding part has a length of about 170 amino acids, so that the expressed fusion protein has an overall length of about 190 amino acids. This fusion protein is expressed, secreted in soluble form in the medium, and purified by affinity chromatography. The authors reported that with this method it might be possible to produce recombinant protein in an amount of hundreds of milligrams per liter of culture. However, this methodology is limited due to alternative processing within the signal peptide sequence and several post-translational modifications of the fused proteins as well as of the cleaved peptides. Assuming an average molecular weight of an amino acid of 110 Daltons, the desired peptides have a molecular weight of about 2,000 to 5,000 Daltons, whereas the fusion tail has a length of at least 170 amino acids (about 19,000 D), if the IgG binding domains of Staphylococcus aureus protein A is used as such a fusion tail. Therefore, only 10 to 25% of the recombinantly produced protein is the desired peptide.

Further examples and methods for the recombinant production of small peptides via fusion proteins in E.coli are described by Uhlen, M., and Moks, T., Methods Enzymol. 185 (1990) 129-143, T.J.R.: Expression of eukaryotic genes in E. coli. In: Genetic Engineering (Williamson, R. ed.), Academic Press, London, vol. 4, 127-185 (1983); and Kopetzki, E., et al., Clin. Chem. 40 (1994) 688-704. Ningyi, L., et al., Gaojishu Tongxun 10 (2000) 28-31 describe the recombinant expression of p24 gag gene in E.coli.

International Application WO 02/103026 describes a process for the production of an antifusogenic peptide as a fusion peptide of a length of about 14 to 70 amino acids in a prokaryotic host cell, characterized in that, under such conditions that inclusion bodies of said fusion peptide are formed, in said host cell there is expressed a nucleic acid encoding said fusion peptide consisting of said antifusogenic peptide of a length of about 10 to 50 amino acids N-terminally linked to a further peptide of a length of about 4 to 30 amino acids; said host cell is cultivated; said inclusion bodies are formed, recovered and solubilized; said fusion peptide is isolated.

The object of the present invention was to provide a method which enables the recombinant production of a high yield of antifusogenic peptides via the inclusion body route and which is suitable for the large-scale industrial production of such peptides.

The invention therefore provides a process for the recombinant production of an antifusogenic peptide by expression of a nucleic acid encoding said antifusogenic peptide as repeat peptide in a microbial host cell, preferably in a prokaryotic host cell, isolating of inclusion bodies containing said repeat peptide, solubilization of the inclusion bodies, and isolation of the antifusogenic peptide after cleavage, characterized by washing the inclusion bodies with a denaturing agent at a pH value of or below pH 6.5, solubilizing said inclusion bodies containing the repeat peptide at a pH value of at least pH 9 and cleaving said repeat peptide to obtain said antifusogenic peptide.

It was surprisingly found that inclusion bodies of repeats of polypeptides having antifusogenic activity (hereinafter also referred to as fusion polypeptides) are resistant to denaturing conditions at pH values of 6.5 and below. Based on this, it is possible to purify inclusion bodies containing such repeat peptides in a highly effective manner from host cell polypeptides and other host cell-derived impurities by the conditions according to the invention, as most of all substances contained in the inclusion bodies are easily soluble under such denaturing conditions and therefore can be removed by simple washing of the inclusion bodies under such denaturing conditions.

In a subsequent step, the inclusion bodies are solubilized at a pH value of 9 and higher for recovery of the soluble repeat peptide, whereby it is not necessary to add detergents or denaturing agents. After solubilisation, the repeat is cleaved chemically or enzymatically to obtain the desired antifusogenic peptide.

In a preferred embodiment of the invention, a cleavage sequence is located between the antifusogenic peptides of the repeat(s) and the repeat peptide is included in a fusion polypeptide characterized in that the repeats are linked together with said cleavage sequence(s) which are preferably of a length of about 1 to 10 amino acids. The antifusogenic peptide itself has a length of 10 to 100 amino acids, whereby (in regard to stable expression and formation of inclusion bodies) the length of the fusion polypeptide preferably is at least about 50 amino acids.

Therefore the invention provides an extremely simple method for recombinant production of antifusogenic peptides via the inclusion bodies route by simply washing the inclusion bodies and subsequently solubilizing them at different pH values.

### Detailed Description of the Invention

"Antifusogenic" and "anti-membrane fusion" as used herein refer to a peptide's ability to inhibit or reduce the level of fusion events between two or more structures, e.g., cell membranes or viral envelopes or pili relative to the level of membrane fusion which occurs between the structures in the absence of the peptide. Examples hereof are peptidic inhibitors of lentiviruses such as human immunodeficiency virus (HIV), respiratory syncytical virus (RSV), human parainfluenza virus (HPV), measles virus (MEV), and Simian immunodeficiency virus (SIV). Such antifusogenic peptides are derived from C helix of a transmembrane subunit of an envelope fusion protein from a virus of the lentivirus genus and bind to the central coiled coil of the transmembrane subunit of the respective virus.

Especially preferred are HIV-1 antifusogenic peptides, preferably fragments of the C-peptide of gp41. Table 1 describes examples of HIV-1 antifusogenic peptides derived from the C-peptide of gp41. These antifusogenic peptides and fragments thereof are particularly useful in the invention.

**Table 1**

| **Name*** | **Name** | **Amino acid sequence (one-letter code)** | **SEQ ID NO:** |
|---|---|---|---|
| T-1249 | T1357¹⁾ | WQEWEQKITALLEQAQIQQEKNEYELQKLDKWASLWEWF | 1 |
| T-20, DP178 | T680²⁾ | YTSLIHSLIEESQNQQEKNEQELLELDKWASLWNWF | 2 |
| T-118 | RSV118³⁾ | FDASISQVNEKINQSLAFIRKSDELLHNVNAGKST | 3 |
| T-257 | MV257³⁾ | LHRIDLGPPISLERLDVGTNLGNAIAKLEDAKELL | 4 |

| | | | |
|---|---|---|---|
| * for N-terminally acetylated and/or C-terminally amidated peptide; T-20 (synonymous with DP178) and T-1249 are from human immunodeficiency virus type 1 (HIV-1), T-118 is from respiratory syncytial virus (RSV) and T-257 is from measles virus (MV) ¹⁾ WO 99/59615 ²⁾ Rimsky, L.T., et al., J. Virol. 72 (1998) 986-993 ³⁾ Lambert, D.M., Proc. Natl. Acad. Sci. USA 93 (1996) 2186-2191 | | | |

The length of the antifusogenic peptide is not critical. It is however preferred to use lengths of about 10 to 100 amino acids. The length must be sufficient to provide stability against denaturing agents at acidic pH values. The maximum length depends primarily on appropriate handling of the fusion polypeptides during solubilization, cleavage and purification.

In a preferred embodiment of the invention, the repeat peptide according to the invention is linked at its N-terminus with a further peptide of about 4 to 30 amino acids. The purpose of the further peptide is to impart additional properties to the antifusogenic peptide, for example, to improve expression (e.g. an N-terminal fragment of a polypeptide with a high expression rate such as interferon-α-2a, purification (e.g. a His-tag; see, e.g., Zhang, J.-H., et al., Nanjing Daxue Xuebao, Ziran Kexue 36(4) (2000) 515-517) or to allow subsequent N-terminal modification like acetylation or PEGylation.

The further peptide is a short peptidic stretch consisting of at least four amino acids (methionine and three further amino acids for cleavage and/or expression purposes) to about 30 amino acids, preferably from 10 to about 20 amino acids (with regard to the nucleic acid codons level). The length of the further peptide is not critical for the invention. However, it is preferred that the further peptide is very short for improving the yield of the antifusogenic peptide. It is especially preferred that the further peptide consists of an appropriate cleavage site of some amino acids compatible with high level expression or to improve access of cleavage proteases (avoidance of steric hindrance) and/or of some amino acids such as purification or immobilization tags such as a His-tag (for purification means; Hengen, P., Trends Biochem. Sci. 20 (1995) 285-286)) and methionine necessary and encoded by the start codon.

The further peptide according to the invention is used in the fusion peptide also preferably to protect the N-terminus of the antifusogenic peptide during expression, solubilization, purification and peptide modification. Such fusion peptides are especially valuable in a process for the production of N-terminally modified antifusogenic peptides. Such a method involves forming the recombinant polypeptide as a fusion peptide, which fusion part protects the N-terminus. The recombinant fusion peptide can then be reacted with chemical protecting agents to selectively protect reactive side-chain groups. Then the fusion peptide is cleaved with at least one cleavage reagent between the peptide and the fusion part to form an unprotected terminal amino acid reactive group. Thereafter, the unprotected terminal amino acid reactive group can be modified with a chemical modifying agent such as acetic anhydride or acetic N-hydroxysuccinimide ester for N-terminal acetylation. Then the side-chains are selectively deprotected to form a N-terminally modified peptide. Such methods are described, for example, in WO 94/01451; U.S. Patent No. 5,635,371; and U.S. Patent No. 5,656,456.

The further peptide part preferably has such a structure that it facilitates the purification and/or immobilization of the fusion peptide. The further peptide preferably contains for this purpose an "affinity tag" (cf. Pandjaitan, B., et al., Gene 237 (1999) 333-342), such as polyhistidine (about 6 His residues) or the like. In addition, the further peptide contains preferably an N-terminal methionine (encoded by ATG) and C-terminally one or more amino acids encoding for a cleavage site.

Especially preferred further peptides according to the invention are N-terminal fragments of human interferon-α-2a, preferably with an included His-tag (amino acids in standard one-letter code):
MCDLPQTHSLGSR (SEQ ID NO:5)
MSDLPQTHSLGSR (SEQ ID NO:6)
MSDLPQTHHHHHHSLGSR (SEQ ID NO:7)

According to the invention, the fusion polypeptides contain one or more cleavage sites which can be cleaved enzymatically after solubilization of the inclusion bodies with a specifically cleaving proteinase (restriction proteinase) or by chemical means. The proteinase is selected, taking into consideration the amino acid sequence of the antifusogenic peptide to be produced. Care must be taken that, if possible, the recognition/cleavage sequence of the restriction proteinase does not occur in the antifusogenic peptide, and preferably also not in the further peptide, i.e., it should only occur in the cleavage region (linker region). Suitable specifically cleaving endoproteinases are, for example, Factor Xa, thrombin, subtilisin, BTN variant of subtilisin, ubiquitin protease, rennin, enterokinase, collagenase, precision protease, trypsin, chymotrypsin, endoproteinase Lys-C, kallikrein (Carter, P.: In: Ladisch, M.R.; Willson, R.C.; Painton, C.C.; Builder, S.E., eds., Protein Purification: From Molecular Mechanisms to Large-Scale Processes; ACS Symposium Series No. 427, American Chemical Society, pp. 181-193 (1990)), TEV proteinase (Parks, T.D., et al., Anal. Biochem. 216 (1994) 413-417), IgA protease (Pohlner, J., et al., Nature 325 (1987) 458-462), Kex2p proteinase (EP-A 0 467 839) or S. aureus V8 proteinase.

The sequence of the further peptide may preferably exploit other design strategies which promote efficient cleavage in the preselected cleavage environment. Particularly if the preselected cleavage agent is an endopeptidase, it is preferred that the further peptide is soluble in aqueous environments. Amino acids having charged side-groups and hydrophilic properties are, therefore, preferably included in the further peptide to promote solubility or any other amino acids which promote the access of cleavage proteinases. Such amino acids are, for example, Glu and Asp (anionic), Arg and Lys (cationic), and Ser and Thr (neutral, hydrophilic). If arginine and/or lysine is used, it must be taken into account that arginine and lysine constitute the trypsin cleavage site.

The cleavage site is typically selected so that cleavage results in free and desired antifusogenic peptides without additional sequences (e.g., trypsin cleaves after Arg and then Arg is removed by carboxypeptidase B). Therefore, the cleavage site is located at one end of each repeat, preferably at the N-terminus of the peptide repeat. This is also preferred in the case where the fusion polypeptide does not contain a further peptide as defined above, as such cleavage site may be used for intermediate protection of the N-terminus during chemical modification of the peptide. Chemical and enzymatic cleavage sites and the corresponding agents used to effect cleavage of a peptide bond close to one of the sites are described, for example, in WO 92/01707 and WO 95/03405.

Examples for cleavage enzymes and the cleavage sequence are described in Table 2 below:

**Table 2**

| **Enzyme** | **Cleavage sequence¹⁾** | **SEQ ID NO:** |
|---|---|---|
| Enterokinase | DDDDK | 8 |
| Factor Xa | IEGR | 9 |
| Thrombin | GPR | 10 |
| Ubiquitin | RGG | |
| Rennin | HPFHL-LVY | 11 |
| Trypsin | K or R | |
| Chymotrypsin | F or Y or W | |
| Clostripain | R | |
| LysC Endoprotease | K | |
| S. aureus V8 | E | |

**Chemical cleavage:**

| **Cleavage substance** | **Cleavage sequence¹⁾** |
|---|---|
| BrCN | M |
| BNPS-skatole | W |
| 2-Nitro-5-thiocyanobenzoate | C |

| | |
|---|---|
| ¹⁾ Amino acid(s) in one-letter code. | |

Trypsin is preferably used, which specifically cleaves proteins and peptides at the C-terminal end of arginine and lysine. Such enzymes are known, for example, from porcine, bovine or human pancreas or recombinant yeasts or E.coli (WO 99/10503). Trypsin is particularly suitable for producing the desired polypeptides, if the lysine residues are protected.

The peptide sequence which can be cleaved by an endoproteinase is understood within the sense of the present invention as a short-chain peptide sequence which is preferably composed of 1 to 20 amino acids, preferably 1 to 3 amino acids, and contains a C-terminal cleavage site for the desired endoproteinase. This further peptide preferably additionally contains a combination of several amino acids (first part) between the N-terminal end and the desired endoproteinase recognition sequence, preferably selected from hydrophilic amino acids such as Gly, Thr, Ser, Ala, Pro, Asp, Glu, Arg and Lys. An amino acid stretch in which two to eight of these additional amino acids are the negatively charged amino acids Asp and/or Glu is preferably used as the first part.

Cleavage is also possible using BrCN (chemical cleavage) as long as the antifusogenic peptide does not contain methionine.

In a further preferred embodiment of the invention, the antifusogenic peptide contains a glycine at its C-terminus. This glycine is useful for the purpose of subsequent enzymatic C-terminal amidation (Bradbury, A.F., and Smyth, D.G., Trends Biochem. Sci.16 (1991) 112-115).

Therefore, the elements of the fusion polypeptides preferably are
- antifusogenic peptide,
- cleavage site,
- further peptide for facilitating expression and/or purification,
- glycine at the C-terminus.

The cleavage sites are necessary for the release of the antifusogenic peptide from the other elements of the fusion polypeptide. Therefore the cleavage sites are located between the antifusogenic peptides in the fusion polypeptide, preferably as an N-terminal protecting group.

According to the invention, an antifusogenic peptide repeat is overexpressed in microorganisms, such as prokaryotes, under conditions whereby insoluble protein inclusion bodies containing said peptide and other polypeptides are formed. The properties according to the invention are already found with a single repeat (two identical antifusogenic peptide sequences) of the antifusogenic peptide. It is, however, also possible to use fusion polypeptides having more than one repeat, i.e., five, ten or up to twenty identical antifusogenic peptide sequences. The fusion peptide to be produced must have a sufficient length for stable expression and for the formation of inclusion bodies in the host cell. Usually, genes encoding at least about 80 amino acids, preferably >100 amino acids, are expressed in a stable manner, are not degraded and are precipitated as inclusion bodies. There is no real upper limit of the repeats. However, to express genes encoding more than 3,000 amino acids is difficult and uncommon in nature.

Escherichia, Salmonella, Streptomyces or Bacillus are for example suitable as prokaryotic host organisms. Yeasts (e.g., Saccharomyces, Pichia, Hansenula, Kluyveromyces, Schizosaccharomyces) are preferred eukaryotic host organisms. For the production of the fusion peptides according to the invention the microorganisms are transformed in the usual manner with the vector which contains the DNA coding for the peptide and subsequently fermented in the usual manner.

Inclusion bodies are found in the cytoplasm and contain the repeat peptide in an aggregated form insoluble in water. Usually, such proteins of inclusion bodies are in a denatured form (e.g., randomly linked disulfide bridges). These inclusion bodies are separated from other cell components, for example by centrifugation after cell lysis.

According to the invention, the inclusion bodies are washed under denaturing conditions at a pH value below 6.5, preferably at about pH 3 to 5. Such denaturing conditions surprisingly do not solubilize the fusion polypeptide to a considerable extent but are able to solubilize a lot of other host cell-derived impurities including polypeptides. Such denaturing agents are well known in the state of the art and are, for example, highly concentrated solutions of guanidinium hydrochloride (e.g. about 6 mol/l) or urea (e.g. about 8 mol/l). The denaturing agent is preferably used as a buffered solution.

After washing, the inclusion bodies are solubilized at alkaline pH values of about pH 9 or higher, preferably without adding of detergents or denaturing agents. It was, in addition, surprisingly found that such alkaline conditions are able to solubilize the fusion polypeptides in a sufficient manner. After solubilization, the fusion polypeptide can be cleaved to recover the antifusogenic peptide.

After solubilization, the fusion peptide or antifusogenic peptide can be purified in a simple fashion, for example by size exclusion chromatography, ion exchange chromatrography or reversed phase chromatography.

All further steps in the process for the construction of suitable expression vectors and for the gene expression are state of the art and familiar to a person skilled in the art. Such methods are described for example in Sambrook et al., Molecular Cloning: A Laboratory Manual (1989), Cold Spring Harbor Laboratory Press, New York, USA.

There exist a large number of publications which describe the recombinant production of proteins in microorganisms/prokaryotes via the inclusion bodies route. Examples of such reviews are Misawa, S., et al., Biopolymers 51 (1999) 297-307; Lilie, H., Curr. Opin. Biotechnol. 9 (1998) 497-501; Hockney, R.C., Trends Biotechnol. 12 (1994) 456-463.

The peptides according to the invention are overexpressed in microorganisms/prokaryotes. Overexpression leads to the formation of inclusion bodies. Methionine encoded by the start codon and mentioned in the examples above is mainly removed during the expression/translation in the host cell. General methods for overexpression of proteins in microorganisms/prokaryotes have been well-known in the state of the art for a long time. Examples of publications in the field are Skelly, J.V., et al., Methods Mol. Biol. 56 (1996) 23-53; Das, A., Methods Enzymol. 182 (1990) 93-112; and Kopetzki, E., et al., Clin. Chem. 40 (1994) 688-704.

Overexpression in prokaryotes means expression using optimized expression cassettes (U.S. Patent No. 6,291,245) with promoters such as the tac or lac promoter (EP-B 0 067 540). Usually, this can be performed by the use of vectors containing chemical inducible promoters or promoters inducible via shift of temperature. One of the useful promoters for E.coli is the temperature-sensitive λPL promoter (cf. EP-B 0 041 767). A further efficient promoter is the tac promoter (cf. U.S. Patent No. 4,551,433). Such strong regulation signals for prokaryotes such as E.coli usually originate from bacteria-challenging bacteriophages (see Lanzer, M., et al., Proc. Natl. Acad. Sci. USA 85 (1988) 8973-8977; Knaus, R., and Bujard, H., EMBO Journal 7 (1988) 2919-2923; for the λT7 promoter: Studier, F.W., et al., Methods Enzymol. 185 (1990) 60-89); for the T5 promoter: EP-A 0 186 069; Stüber, D., et al., System for high-level production in Escherichia coli and rapid application to epitope mapping, preparation of antibodies, and structure-function analysis; In: Immunological Methods IV (1990) 121-152).

By the use of such overproducing prokaryotic cell expression systems the peptides according to the invention are produced at levels at least comprising 10% of the total expressed protein of the cell, and typically 30-40%, and occasionally as high as 50%.

"Inclusion bodies" (IBs) as used herein refer to an insoluble form of polypeptides recombinantly produced after overexpression of the encoding nucleic acid in microorganisms/prokaryotes. This phenomenon is widely known in the state of the art and is reviewed, for example, by Misawa S., and Kumagai, I., Biopolymers 51 (1999) 297-307); Guise, A.D., et al., Mol. Biotechnol. 6 (1996) 53-64; and Hockney et al., Trends Biotechnol. 12 (1994) 456-463.

Solubilization of the inclusion bodies is preferably performed by the use of aqueous solutions with pH values of about 9 or higher. Most preferred is a pH value of 10.0 or higher. It is not necessary to add detergents or denaturing agents for solubilization. The optimized pH value can be easily determined according to Example 7. It is obvious that there exists an optimized pH range as strong alkaline conditions might denature the polypeptides. This optimized range is found between pH 9 and pH 12.

Nucleic acids (DNA) encoding the fusion peptide can be produced according to the methods known in the state of the art. It is further preferred to extend the nucleic acid sequence with additional regulation and transcription elements, in order to optimize the expression in the host cell. A nucleic acid (DNA) that is suitable for the expression can preferably be produced by chemical synthesis. Such processes are familiar to a person skilled in the art and are described for example in Beattie, K. L., and Fowler, R. F., Nature 352 (1991) 548-549; EP-B 0 424 990; Itakura, K., et al., Science 198 (1977) 1056-1063. It may also be expedient to modify the nucleic acid sequence of the peptides according to the invention.

Such modifications are, for example:
- modification of the nucleic acid sequence in order to introduce various recognition sequences of restriction enzymes to facilitate the steps of ligation, cloning and mutagenesis;
- modification of the nucleic acid sequence to incorporate preferred codons for the host cell;
- extension of the nucleic acid sequence with additional regulation and transcription elements in order to optimize gene expression in the host cell.

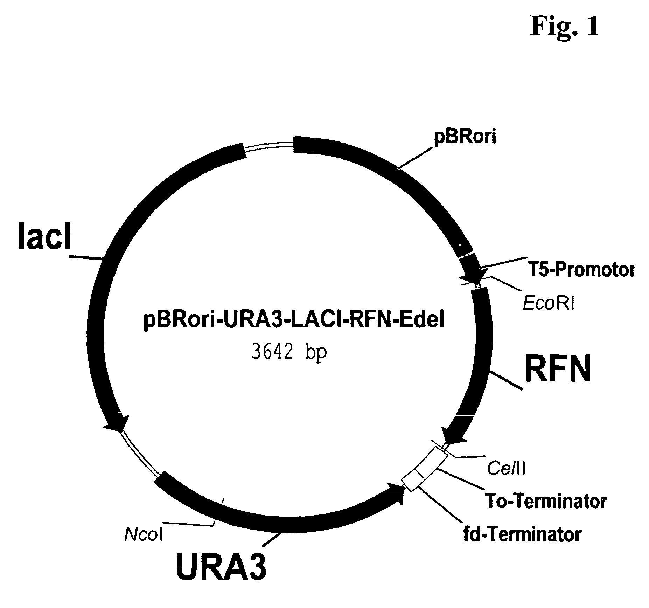

The following examples, references, Fig. 1 and the sequence listing are provided to aid the understanding of the present invention, the true scope of which is set forth in the appended claims. It is understood that modifications can be made in the procedures set forth without departing from the spirit of the invention.

### Description of the Figure:

- **Figure 1**: shows the expression vector pBRori-URA3-LACI-RFN-Edel.

### Starting material

The E.coli host/vector system (E.coli host strain and basic vector) employed for the expression of the multimeric peptide precursor proteins according to the invention is described in US Patent No. 6,291,245.

### General Methods

Recombinant DNA technique

Standard methods were used to manipulate DNA as described in Sambrook, J., et al., Molecular cloning: A laboratory manual; Cold Spring Harbor Laboratory Press, Cold Spring Harbor, New York, 1989. The molecular biological reagents were used according to the manufacturer's instructions.

### Protein determination

The protein concentration for the multimeric T-repeat fusion protein was determined by determining the optical density (OD) at 280 nm, using the molar extinction coefficient calculated on the basis of the amino acid sequence [T-repeat: ε = 148680 cm²/mol; IFN-α-2a: ε = 18020 cm²/mol; F9a = 44650 cm²/mol].

### Example 1

### Synthesis of the T-repeat fusion gene

### 1.1 Gene design of the T-repeat fusion gene

The artificial T-repeat fusion gene encodes a fusion protein of 217 amino acids having a molecular weight of 27,242 D. The fusion protein is composed of the N-terminal 13 amino acids of human interferon-α-2a (MCDLPQTHSLGSR (SEQ ID NO:5); carrier peptide) and 5 copies of the inhibitory HIV peptide T-1357 (WQEWEQKITALLEQAQIQQEKNEYELQKLDKWASLWEWF (SEQ ID NO:1), target peptide), which are connected via trypsin-cleavable peptide linkers (GR).

In order to insert the T-repeat structural gene into the E.coli expression plasmid pBRori-URA3-LacI-RFN-Edel (production and description: see Example 2), a synthetic ribosomal RBSII binding site and a singular EcoRI cleavage site were inserted upstream at the 5' end and a singular CelII restriction endonuclease cleavage site upstream at the 3' end.

### 1.2 Gene synthesis of the T-repeat fusion gene

The RBSII T-repeat gene which is about 690 bp long and flanked by a singular EcoRI and CelII restriction endonuclease cleavage site was prepared from oligonucleotides by chemical synthesis. The double-stranded RBSII T-repeat gene was assembled by annealing and ligation of the oligonucleotides and subsequently cloned as an EcoRI/CelII fragment of a length of 691 bp into an E.coli plasmid. The desired plasmid was designated pT-repeat. The predetermined DNA sequence of the cloned RBSII T-repeat gene was confirmed by DNA sequencing.

### Example 2

### Construction of the E.coli expression plasmids

### 2.1 Construction of the starting plasmid pBRori-URA3-LacI-RFN-Edel

The plasmid pBRori-URA3-LacI-RFN-Edel is a vector for the expression of interferon-α-2a (IFN-α-2a) in E.coli. It is based on the IFN-α-2b expression plasmid OripBR-URA3-EK-IFN (U.S. Patent No. 6,291,245). pBRori-URA3-LacI-RFN-Edel differs from OripBR-URA3-EK-IFN by an additionally present lacI repressor gene and an IFN-α-2a gene instead of an IFN-α-2b gene. IFN-α-2a and IFN-α-2b differ only by one amino acid at position 21 (Lys21Arg exchange). The lacI repressor gene comes from plasmid pUHA1 (Stüber, D., et al., System for high-level production in Escherichia coli and rapid application to epitope mapping, preparation of antibodies, and structure-function analysis; In: Immunological Methods IV (1990) 121-152). It was amplified by polymerase chain reaction (PCR) according to the method described by Mullis, K.B., and Faloona, F.A., in Methods Enzymol. 155 (1987) 335-350, using the primers N1 (SEQ ID NO:12) and N2 (SEQ ID NO:13) and subsequently ligated as a NotI fragment of a length of about 1210 bp into the singular NotI cleavage site of OripBR-URA3-EK-IFN.

### 2.2 Construction of the expression vector pBRori-URA3-LacI-T-repeat

The T-repeat gene was isolated as an EcoRI/CelII fragment of a length of about 690 bp from the plasmid pT-repeat (see Example 1.2) and subsequently ligated into the ca. 3.1 kbp long pBRori-URA3-LacI-RFN vector fragment digested with EcoRI and CelII. The desired plasmid pBRori-URA3-LacI-T-repeat was identified by restriction mapping and subcloned T-repeat gene was verified again by DNA sequencing.

### Example 3

### Expression of the T-repeat gene in E.coli

For the expression of the fusion gene according to the invention there was employed an E.coli host/vector system which enables an antibiotic-free plasmid selection by complementation of an E.coli auxotrophy (PyrF) (U.S. Patent No. 6,291,245).

### 3.1 Transformation and cell culturing by complementation of a pyrF auxotrophy in selective medium

In order to express the T-repeat gene, an E.coli K12 strain [designated UT5600 (ΔpyrF)] was transformed with the expression plasmid pBRori-URA3-LacI-T-repeat described in Example 2.2. The transformed UT5600(ΔpyrF)/pBRori-URA3-Lacl-T-repeat cells were first grown at 37°C on agar plates and subsequently in a shaking culture in M9 minimal medium containing 0.5% casamino acids (Difco) up to an optical density at 550 nm (OD₅₅₀) of 0.6-0.9 and subsequently induced with IPTG (1-5 mmol/l final concentration). After an induction phase of 4-16 hours at 37°C, the cells were harvested by centrifugation, washed with 50 mmol/l potassium phosphate buffer, pH 6.5, and stored at -20°C until further processing.

### 3.2 Expression analysis

For expression analysis cell pellets from 3 OD₅₅₀ₙₘ units (1 OD₅₅₀ₙₘ = 1 ml cell suspension with an OD at 550 nm of 1) of centrifuged culture medium were resuspended in 0.25 ml 10 mmol/l potassium phosphate buffer, pH 6.5, and the cells were lysed by ultrasonic treatment (two pulses of 30 s at 50% intensity). The insoluble cell components were sedimented (14,000 rpm, 5 min) and the supernatant was admixed with 1/5 volumes (vol) 5xSDS sample buffer (1xSDS sample buffer: 50 mmol/l Tris-HCl, pH 6.8, 1% SDS, 50 mmol/l DTT, 10% glycerol, 0.001% bromophenol blue). The insoluble cell debris fraction (pellet) was resuspended in 0.3 ml 1xSDS sample buffer, the samples were incubated for 5 min at 95°C and again centrifuged. Subsequently, the proteins were separated by SDS polyacrylamide gel electrophoresis (PAGE) (Laemmli, U.K., Nature 227 (1970) 680-685) and stained with Coomassie Brilliant Blue R dye.

The synthesized T-repeat fusion protein was homogeneous and was found exclusively in the insoluble cell debris fraction in the form of insoluble protein aggregates, the so-called inclusion bodies (IBs). The expression yield was comparable within the scope of the measurement accuracy in all clones and was between 30-60% relative to the total E.coli protein.

### Example 4

### 10 l high cell density fermentations of E.coli for the recombinant production of T-repeat

### Preculture

In order to prepare the preculture, 300 ml M9plus medium (M9 medium supplemented with 0.5% casamino acids and 0.9 g/l Trp, Pro and Leu each) was inocculated with 1 ml of a glycerol stock of E.coli UT5600ΔpyrF pBRori-URA3-lacI-T-repeat in a 1000 ml Erlenmeyer flask. The culture was incubated for about 6 hours at 37°C on an excenter shaker with 150 rpm until an OD₅₇₈ₙₘ of 3.0 was reached.

### 10 l Fed-batch main fermentation

At the beginning of fermentation, the preculture was transferred into the 10 liter fermenter. The main culture was grown in defined M9 salt medium containing 1.4% glycerol instead of glucose, 2% casamino acids and 0.1% of the amino acids Trp, Leu and Pro each, up to an OD₅₇₈ₙₘ of 20. Subsequently, feeding of the culture with a glycerol yeast dosage (stock solution: 30% yeast extract and 33% glycerol) was started, the flow rate of which was varied between 0.8 and 3.5 ml/min depending on the development of the pH value of the culture, thereby avoiding any further addition of correction aids (H₃PO₄, KOH). The pH was maintained at pH 7.0, the pO₂ value was held at 50% by controlling the rpm.

At an OD₅₇₈ₙₘ of 70 1.5 mmol/l IPTG was added and the gene/protein expression was induced. The total fermentation took about 36 hours and was terminated at an OD₅₇₈ₙₘ of 160-180.

### Harvesting the biomass

The content of the fermenter was centrifuged with a flowthrough centrifuge (13,000 rpm, 131/h) and the harvested biomass was stored at -20°C until further processing.

### Example 5

### 5.1 Standard methods: cell lysis and preparation of IBs

200 g E.coli cells (wet weight) were suspended in 110.1 mol/l Tris-HCl, pH 7.0, at 0°C, 300 mg lysozyme were added and incubated for 20 minutes at 0°C. Subsequently, the cells were completely lysed mechanically by means of high pressure dispersion and the DNA was digested for 30 minutes at 25°C by adding 2 ml 1 mol/l MgCl₂ and 10 mg DNAse. Thereafter, 500 ml 60 mmol/l EDTA, 6% Triton X-100 and 1.5 mol/l NaCl, pH 7.0 were admixed with the lysis solution and incubated for another 30 minutes at 0°C. Subsequently, the insoluble components (cell debris and IBs) were sedimented by centrifugation.

The pellet was suspended in 11 0.1 mol/l Tris-HCl, 20 mmol/l EDTA, pH 6.5, incubated for 30 minutes at 25° and the IB preparation was isolated by centrifugation.

### 5.2 Methods according to the invention: cell lysis and preparation of IBs

200 g E.coli cells (wet weight) were suspended in 110.1 mol/l potassium phosphate buffer, pH 5.0, 300 mg lysozyme were added and incubated for 20 minutes at 0°C. Subsequently, the cells were completely lysed mechanically by means of high pressure dispersion, the DNA was digested for 30 minutes at 25°C by adding 2 ml 1 mol/l MgCl₂ and 10 mg DNAse and the insoluble components (cell debris and lBs) were sedimented by centrifugation.

Thereafter, the pellet was washed twice with 0.1 mol/l potassium phosphate buffer, pH 5.0. To this end, the pellet was suspended twice in 11 0.1 mol/l potassium buffer, pH 5.5, incubated at 25°C for 30 minutes while stirring and isolated by centrifugation.

### 5.3 Methods according to the invention: cell lysis and preparation of high purity IBs

200 g E.coli cells (wet weight) were suspended in 110.1 mol/l potassium phosphate buffer, pH 5.0, at 0°C, 300 mg lysozyme were added and incubated for 20 minutes at 0°C. Subsequently, the cells were completely lysed mechanically by means of high pressure dispersion, the DNA was digested for 30 minutes at 25°C by adding 2 ml 1 mol/l MgCl₂ and 10 mg DNAse and the insoluble components (cell debris and IBs) were sedimented by centrifugation.

Thereafter, the pellet was washed twice with 5.5 mol/l guanidinium hydrochloride (and 8 mol/l urea, respectively) and 10 mmol/l EDTA, pH 3.0). To this end, the pellet was suspended twice in 5.5 mol/l guanidinium hydrochloride (and 8 mol/l urea, respectively) and 10 mmol/l EDTA, pH 3.0, incubated at 25°C for 10-30 minutes while stirring and isolated by centrifugation.

Thereafter, the pellet was washed two to three times with 0.1 mol/l potassium phosphate buffer, pH 5.0. To this end, the pellet was suspended two to three times in 11 0.1 mol/l potassium phosphate buffer, pH 5.5, incubated at 25°C for 30 minutes while stirring and isolated by centrifugation.

### Example 6

### Solubility of T-repeat IBs in comparison with "standard" IBs (IFN-a-2a and F9a) in the presence of denaturing agents in the acidic range

The IBs to be tested [IFN-α-2a: prepared as described in Examples 2.1, 3.1, 4 and 5.1, whereby however UT5600(ΔpyrF)/pBRori-URA3-LacI-RFN-Edel cells were used instead of UT5600(ΔpyrF)/pBRori-URA3-LacI-T-repeat cells; F9a: prepared as described in WO 97/47737; T-repeat: prepared as described in Examples 2.2, 3.1, 4 and 5.2] were resuspended at a concentration of 1 g/20 ml each in
a) 5.5 mol/l guanidinium hydrochloride and 10 mmol/l EDTA, pH 3.0 and
b) 8 mol/l urea and 10 mmol/l EDTA, pH 3.0
at room temperature while stirring.

After 12-24 hours, a 200 µl sample was taken in each case, the insoluble components were sedimented by centrifugation (Eppendorf 5415 centrifuge, 14.000 rpm, 10 min) and 80 µl supernatant were mixed with 20 µl 5xSDS sample buffer (1xSDS sample buffer: 50 mmol/l Tris-HCl, pH 6.8, 1% SDS, 50 mmol/l DTT, 10% glycerol, 0.001% bromophenol blue). The supernatants containing 5.5 mol/l guanidinium hydrochloride were dialyzed against 8 mol/l urea prior to SDS PAGE. The insoluble pellet was washed once with 200 µl potassium phosphate buffer, pH 5.0 and subsequently resuspended in 250 µl 1xSDS sample buffer. All samples were incubated at 95°C for 5 minutes before the proteins were separated by means of SDS polyacrylamide gel electrophoresis (PAGE) and stained with Coomassie Brilliant Blue R dye. Thereafter, the relevant gel bands were analyzed densitometrically for the determination of the polypeptide content (Table 3).

**Table 3a**

| **5.5 mol/l guanidinium hydrochloride, 10 mmol/l EDTA, pH 3** | | |
|---|---|---|
| **Protein** | **Supernatant (soluble) %** | **Pellet (insoluble) %** |
| **T-Repeat** | 3 | 97 |
| **IFN-α-2a** | 94 | 6 |
| **F9a** | 89 | 11 |

**Table 3b**

| **8 mol/l urea, 10 mmol/l EDTA, pH 3** | | |
|---|---|---|
| **Protein** | **Supernatant (soluble) %** | **Pellet (insoluble) %** |
| **T-Repeat** | 9 | 91 |
| **IFN-α-2a** | 98 | 2 |
| **F9a** | 90 | 10 |

### Example 7

### Solubility of T-repeat IBs in comparison with "standard" IBs (IFN-α-2a and F9a) in aqueous buffers in the alkaline range

The IFN-α-2a, F9a- and T-repeat IBs to be solubilized (production in accordance with the description in Example 6) were incubated in
a) 50 mmol/l Tris HCl and
b) 50 mmol/l NaHCO₃, respectively,

at the pH values to be tested (8.0, 9.0, 10.0, 11.0 and 12.0) at room temperature while stirring. After 4 hours, a 200 µl sample was taken in each case and the insoluble components were sedimented by centrifugation (14.000 rpm, 15 min). 80 µl supernatant were mixed with 20 µl 5xSDS sample buffer (1xSDS sample buffer: 50 mmol/l Tris-HCl, pH 6.8, 1% SDS, 50 mmol/l DTT, 10% glycerol, 0.001% bromophenol blue). The insoluble pellet was washed once with 200 µl 50 mmol/l potassium phosphate buffer, pH 6.5 and resuspended in 250 µl 1xSDS sample buffer. The samples were incubated at 95°C for 5 minutes before the proteins were separated by means of SDS polyacrylamide gel electrophoresis (PAGE) and stained with Coomassie Brilliant Blue R dye. Thereafter, the relevant gel bands were analyzed densitometrically for the determination of the polypeptide content (Table 4).

**Table 4a**

| **IFN-α-2a** | | | | |
|---|---|---|---|---|
| **pH** | **50 mmol/l Tris HCl** | | **50 mmol/l NaHCO₃** | |
| | **Supernatant (soluble) %** | **Pellet (insoluble) %** | **Supernatant (soluble) %** | **Pellet (insoluble) %** |
| 8.0 | 0.5 | 99.5 | - | - |
| 9.0 | 1 | 99 | 0.7 | 99.3 |
| 10.0 | - | - | 1.3 | 98.7 |
| 11.0 | - | - | 8.0 | 92 |
| 12.0 | - | - | 5.3 | 94.7 |

**Table 4b**

| **F9a** | | | | |
|---|---|---|---|---|
| **pH** | **50 mmol/l Tris HCl** | | **50 mmol/l NaHCO₃** | |
| | **Supernatant (soluble) %** | **Pellet (insoluble) %** | **Supernatant (soluble) %** | **Pellet (insoluble) %** |
| 8.0 | 0 | 100 | - | - |
| 9.0 | 0 | 100 | 0.3 | 99.7 |
| 10.0 | - | - | 0.3 | 99.7 |
| 11.0 | - | - | 2.2 | 97.8 |
| 12.0 | - | - | 43 | 57 |

**Table 4c**

| **T-Repeat** | | | | |
|---|---|---|---|---|
| **pH** | **50 mmol/l Tris HCl** | | **50 mmol/l NaHCO₃** | |
| | **Supernatant (soluble) %** | **Pellet (insoluble) %** | **Supernatant (soluble) %** | **Pellet (insoluble) %** |
| 8.0 | 35 | 65 | 5 | 95 |
| 9.0 | 72 | 28 | 49 | 51 |
| 10.0 | 92 | 8 | 64 | 36 |
| 11.0 | - | - | 97 | 3 |
| 12.0 | - | - | 97 | 3 |

### Example 8

### 8.1 Standard methods: solubilization of IBs under denaturing conditions

20 g IB pellet (wet weight) were suspended in 200 ml 50 mmol/l Tris-HCl buffer, 6 mol/l guanidinium hydrochloride (and 8 mol/l urea, respectively), 10 mmol/l EDTA, pH 7.0 by being stirred for two hours at 25°C. The insoluble components were separated by centrifugation and the clear supernatant was further processed.

### 8.2 Methods according to the invention: solubilization of IBs in aqueous slightly alkaline buffers

10 g IB pellet (wet weight) were suspended in 200 ml 50 mmol/l Tris-HCl buffer, pH 9 (and 50 mmol/l NaHCO₃ buffer, pH 9-10, respectively) by being stirred for 4-16 hours at 25°C. The insoluble components were separated by centrifugation and the clear supernatant was further processed [optionally after derivatization of the primary amino groups of the T-repeat fusion protein (ε-amino group of lysine) with citraconic acid anhydride] by enzymatic cleavage with trypsin.

### List of References

Andersson, L., et al., Biopolymers 55 (2000) 227-250
Arthur, L.O., et al., Science 258 (1992) 1935-1938
Beattie, K. L., and Fowler, R. F., Nature 352 (1991) 548-549
Bradbury, A.F., and Smyth, D.G., Trends Biochem. Sci. 16 (1991) 112-115
Bullough, P.A., et al., Nature 371 (1994) 37-43
Cao, J., et al., J. Virol. 67 (1993) 2747-2755
Carr, C.M., and Kim, P.S., Cell 73 (1993) 823-832
Carter, P.: In: Ladisch, M.R.; Willson, R.C.; Painton, C.C.; Builder, S.E., eds., Protein Purification: From Molecular Mechanisms to Large-Scale Processes; ACS Symposium Series No. 427, American Chemical Society, pp. 181-193 (1990)
Chan, D.C., et al., Proc. Natl. Acad. Sci. USA 95 (1998) 15613-15617
Chan, D.C., et al., Cell 89 (1997) 263-273
Chan, D.C., et al., Cell 93 (1998) 681-684
Chen, C.H., et al., J. Virol. 69 (1995) 3771-3777
Das, A., Methods Enzymol. 182 (1990) 93-112
EP-A 0 186 069
EP-A 0 467 839
EP-B 0 424 990
EP-B 0 041 767
EP-B 0 067 540
Gallaher, W.R., et al., Aids Res. Hum. Retrovirus 5 (1989) 431-440
Guise, A.D., et al., Mol. Biotechnol. 6 (1996) 53-64
Hammarskjöld, M.-L., et al., Biochim. Biophys. Acta 989 (1989) 269-280
Hengen, P., Trends Biochem. Sci. 20 (1995) 285-286
Hockney, R.C., Trends Biotechnol. 12 (1994) 456-463
Itakura, K., et al., Science 198 (1977) 1056-1063
Jakubke, H.D., Peptide, Spektrum Akad. Verlag, Heidelberg (1996), p. 198
Jones, J.H., J. Pept. Sci. 6 (2000) 201-207
Judice, J. K., et al., Proc. Natl. Acad. Sci. USA 94 (1997) 13426-13430
Kilby, J.M., et al., Nature Medicine 4 (1998) 1302-1307
Knaus, R., and Bujard, H., EMBO Journal 7 (1988) 2919-2923
Kopetzki, E., et al., Clin. Chem. 40 (1994) 688-704
Laemmli, U.K., Nature 227 (1970) 680-685
Lambert, D.M., et al., Proc. Natl. Acad. Sci. USA 93 (1996) 2186-2191
Lanzer, M., et al., Proc. Natl. Acad. Sci. USA 85 (1988) 8973-8977
Lawless, M.K., et al., Biochemistry 35 (1996) 13697-13708
Lepage, P., et al., Analytical Biochemistry 213 (1993) 40-48
Lilie, H., Curr. Opin. Biotechnol. 9 (1998) 497-501
Maddon, P.J., et al., Cell 47 (1986) 333-348
Malashkevich, V.N., et al., Proc. Natl. Acad. Sci. USA 95 (1998) 9134-9139
McDougal, J.S., et al., Science 231 (1986) 382-385
Mergler, M., et al., Tetrahedron Letters 29 (1988) 4005-4008 and 4009-4012
Misawa, S., et al., Biopolymers 51 (1999) 297-307
Mullis, K.B. und Faloona, F.A., Methods Enzymol. 155 (1987) 335-350
Ningyi, L., et al., Gaojishu Tongxun 10 (2000) 28-31
Pandjaitan, B., et al., Gene 237 (1999) 333-342
Parks, T.D., et al., Anal. Biochem. 216 (1994) 413-417
Pohlner, J., et al., Nature 325 (1987) 458-462
Rimsky, L.T., et al., J. Virol. 72 (1998) 986-993
Root, M.J., et al., Science 291 (2001) 884-888
Sambrook et al., Molecular Cloning: A Laboratory Manual (1989), Cold Spring Harbor Laboratory Press, New York, USA
Skelly, J.V., et al., Methods Mol. Biol. 56 (1996) 23-53
Stuber, D., et al., System for high-level production in Escherichia coli and rapid application to epitope mapping, preparation of antibodies, and structure-function analysis; In: Immunological Methods IV (1990) 121-152
Studier, F.W., et al., Methods Enzymol. 185 (1990) 60-89
Tan, K., et al., Proc. Natl. Acad. Sci. USA 94 (1997) 12303-12308
Turner, B.G., and Summers, M.F., J. Mol. Biol. 285 (1999) 1-32
U.S. Patent No. 4,551,433
U.S. Patent No. 5,464,933
U.S. Patent No. 5,635,371
U.S. Patent No. 5,656,456
U.S. Patent No. 5,656,480
U.S. Patent No. 6,013,263
U.S. Patent No. 6,017,536
U.S. Patent No. 6,020,459
U.S. Patent No. 6,060,065
U.S. Patent No. 6,093,794
U.S. Patent No. 6,291,245
Uhlen, M., and Moks, T., Methods Enzymol. 185 (1990) 129-143
Weissenhorn, W., et al., Nature 387 (1997) 426-430
Wild, C.T., et al., Proc. Natl. Acad. Sci. USA 91 (1994) 9770-9774
Wilson, I.A., et al., Nature 289 (1981) 366-373
WO 00/69902
WO 02/103026
WO 92/01707
WO 94/01451
WO 95/03405
WO 96/19495
WO 96/40191
WO 99/10503
WO 99/48513
WO 99/59615
Zhang, J.-H., et al., Nanjing Daxue Xuebao, Ziran Kexue 36(4) (2000) 515-517
Zhao, X., et al., Proc. Natl. Acad. Sci. USA 97 (2000) 14172-14177

## Claims

1. Process for the recombinant production of an antifusogenic peptide by expression of a nucleic acid encoding said antifusogenic peptide as repeat peptide in a microbial host cell, isolation of inclusion bodies containing said repeat peptide, solubilization of the inclusion bodies, and isolation of the antifusogenic peptide after cleavage, **characterized by** washing the inclusion bodies with a denaturing agent at a pH value of or below pH 6.5, solubilizing said inclusion bodies containing the repeat peptide at a pH value of at least pH 9 and cleaving said repeat peptide to obtain said antifusogenic peptide.

2. Process according to claim 1, **characterized in that** washing is performed between pH 3 and 5.

3. Process according to claim 1 or 2, **characterized in that** said repeat peptide is cleaved during or after solubilization of said inclusion bodies.

4. A nucleic acid encoding a fusion polypeptide consisting of (in N-terminal to C-terminal direction)
a) an antifusogenic peptide as repeat peptide of at least two identical antifusogenic peptide sequences;
b) a cleavage site located between the antifusogenic peptides.

5. A nucleic acid according to claim 4, **characterized in that** the antifusogenic peptide consists of 10 to 100 amino acids.

6. A nucleic acid according to claim 4 or 5, **characterized in that** the repeat consists of 2 to 20 identical antifusogenic peptide sequences.

7. A preparation of inclusion bodies containing a fusion polypeptide consisting of (in N-terminal to C-terminal direction)
a) an antifusogenic peptide as repeat peptide of a length of about 10 to 100 amino acids;
b) a cleavage site located between the antifusogenic peptides.

## Patentansprüche

1. Verfahren zur rekombinanten Herstellung eines antifusogenen Peptids durch Expression einer Nukleinsäure, die für das antifusogene Peptid codiert, als Repeat-Peptid in einer mikrobiellen Wirtszelle, Isolierung von Einschlusskörpern, die das Repeat-Peptid enthalten, Solubilisieren der Einschlusskörper und Isolierung des antifusogenen Peptids nach Spaltung, **gekennzeichnet durch** Waschen der Einschlusskörper mit einem Denaturierungsmittel bei einem pH-Wert von oder unter pH-Wert 6,5, Solubilisieren der Einschlusskörper, die das Repeat-Peptid enthalten, bei einem pH-Wert von mindestens pH-Wert 9 und Spalten des Repeat-Peptids, um das antifusogene Peptid zu erhalten.

2. Verfahren nach Anspruch 1, **dadurch gekennzeichnet, dass** das Waschen zwischen pH-Wert 3 und 5 durchgeführt wird.

3. Verfahren nach Anspruch 1 oder 2, **dadurch gekennzeichnet, dass** das Repeat-Peptid während oder nach der Solubilisierung der Einschlusskörper gespalten wird.

4. Nukleinsäure, die für ein Fusionspolypeptid codiert, bestehend aus (in N-terminaler nach C-terminaler Richtung)
a) einem antifusogenen Peptid als Repeat-Peptid von mindestens zwei identischen antifusogenen Peptidsequenzen;
b) einer Spaltungsstelle, die zwischen den antifusogenen Peptiden lokalisiert ist.

5. Nukleinsäure nach Anspruch 4, **dadurch gekennzeichnet, dass** das antifusogene Peptid aus 10 bis 100 Aminosäuren besteht.

6. Nukleinsäure nach Anspruch 4 oder 5, **dadurch gekennzeichnet, dass** die Wiederholung aus 2 bis 20 identischen antifusogenen Peptidsequenzen besteht.

7. Präparat von Einschlusskörpern, enthaltend ein Fusionspolypeptid, bestehend aus (in N-terminaler nach C-terminaler Richtung)
a) einem antifusogenen Peptid als Repeat-Peptid mit einer Länge von etwa 10 bis 100 Aminosäuren
b) einer Spaltungsstelle, die zwischen den antifusogenen Peptiden lokalisiert ist.

## Revendications

1. Procédé pour la production recombinante d'un peptide anti-fusogénique par expression d'un acide nucléique codant ledit peptide anti-fusogénique en tant que peptide de répétition dans une cellule hôte microbienne, l'isolation des corps d'inclusion contenant ledit peptide de répétition, la solubilisation des corps d'inclusion et l'isolation du peptide anti-fusogénique après clivage, **caractérisé par** le lavage des corps d'inclusion avec un agent dénaturant à une valeur de pH égale ou inférieure à pH 6,5, la solubilisation desdits corps d'inclusion contenant le peptide de répétition à une valeur de pH d'au moins pH 9 et clivage dudit peptide de répétition pour obtenir ledit peptide anti-fusogénique.

2. Procédé selon la revendication 1, **caractérisé en ce que** le lavage est effectué entre pH 3 et 5.

3. Procédé selon la revendication 1 ou 2, **caractérisé en ce que** ledit peptide de répétition est clivé pendant ou après la solubilisation desdits corps d'inclusion.

4. Acide nucléique codant un polypeptide de fusion consistant (dans la direction N-terminale à C-terminale) en :
a) un peptide anti-fusogénique en tant que peptide de répétition d'au moins deux séquences de peptides anti-fusogéniques identiques ;
b) un site de clivage situé entre les peptides anti-fusogéniques.

5. Acide nucléique selon la revendication 4, **caractérisé en ce que** le peptide anti-fusogénique consiste en 10 à 100 acides aminés.

6. Acide nucléique selon la revendication 4 ou 5, **caractérisé en ce que** la répétition consiste en 2 à 20 séquences de peptides anti-fusogéniques identiques.

7. Préparation de corps d'inclusion contenant un polypeptide de fusion consistant (dans la direction N-terminale à C-terminale) en:
a) un peptide anti-fusogénique en tant que peptide de répétition d'une longueur d'environ 10 à 100 acides aminés ;
b) un site de clivage situé entre les peptides anti-fusogéniques.


### SEQUENCE LISTING

<110> F. HOFFMANN-LA ROCHE AG
<120> Methods for the recombinant production of antifusogenic peptides
<130> 21489 EP1
<140>
   <141>
<150> EP02025618.6
   <151> 2002-11-19
<150> EP03000988.0
   <151> 2003-01-17
<160> 13
<170> PatentIn Ver. 2.1
<210> 1
   <211> 39
   <212> PRT
   <213> Artificial Sequence
<220>
   <223> Description of Artificial Sequence:peptide T1357
<400> 1
   Trp Gln Glu Trp Glu Gln Lys Ile Thr Ala Leu Leu Glu Gln Ala Gln
     1               5                  10                  15
   Ile Gln Gln Glu Lys Asn Glu Tyr Glu Leu Gln Lys Leu Asp Lys Trp
                20                  25                  30
   Ala Ser Leu Trp Glu Trp Phe
            35
<210> 2
   <211> 36
   <212> PRT
   <213> Artificial Sequence
<220>
   <223> Description of Artificial Sequence:peptide T680
<400> 2
   Tyr Thr Ser Leu Ile His Ser Leu Ile Glu Glu Ser Gln Asn Gln Gln
     1               5                  10                  15
   Glu Lys Asn Glu Gln Glu Leu Leu Glu Leu Asp Lys Trp Ala Ser Leu
                20                  25                  30
   Trp Asn Trp Phe
            35
<210> 3
   <211> 35
   <212> PRT
   <213> Artificial Sequence
<220>
   <223> Description of Artificial Sequence:peptide RSV118
<400> 3
   Phe Asp Ala Ser Ile Ser Gln Val Asn Glu Lys Ile Asn Gln Ser Leu
    1               5                  10                  15
   Ala Phe Ile Arg Lys Ser Asp Glu Leu Leu His Asn Val Asn Ala Gly
               20                  25                  30
   Lys Ser Thr
           35
<210> 4
   <211> 35
   <212> PRT
   <213> Artificial Sequence
<220>
   <223> Description of Artificial Sequence:peptide MV257
<400> 4
   Leu His Arg Ile Asp Leu Gly Pro Pro Ile Ser Leu Glu Arg Leu Asp
    1               5                  10                  15
   Val Gly Thr Asn Leu Gly Asn Ala Ile Ala Lys Leu Glu Asp Ala Lys
               20                  25                  30
   Glu Leu Leu
           35
<210> 5
   <211> 13
   <212> PRT
   <213> Artificial Sequence
<220>
   <223> Description of Artificial Sequence:peptide
<400> 5
   Met Cys Asp Leu Pro Gln Thr His Ser Leu Gly Ser Arg
    1               5                  10
<210> 6
   <211> 13
   <212> PRT
   <213> Artificial Sequence
<220>
   <223> Description of Artificial Sequence:peptide
<400> 6
   Met Ser Asp Leu Pro Gln Thr His Ser Leu Gly Ser Arg
   1 5 10
<210> 7
   <211> 18
   <212> PRT
   <213> Artificial Sequence
<220>
   <223> Description of Artificial Sequence:peptide
<400> 7
   Met Ser Asp Leu Pro Gln Thr His His His His His His Ser Leu Gly
   1 5 10 15
   Ser Arg
<210> 8
   <211> 5
   <212> PRT
   <213> Artificial Sequence
<220>
   <223> Description of Artificial Sequence:cleavage sequence
<400> 8
   Asp Asp Asp Asp Lys
   1 5
<210> 9
   <211> 4
   <212> PRT
   <213> Artificial Sequence
<220>
   <223> Description of Artificial Sequence:cleavage sequence
<400> 9
   Ile Glu Gly Arg
   1
<210> 10
   <211> 3
   <212> PRT
   <213> Artificial Sequence
<220>
   <223> Description of Artificial Sequence:cleavage sequence
<400> 10
   Gly Pro Arg
    1
<210> 11
   <211> 8
   <212> PRT
   <213> Artificial Sequence
<220>
   <223> Description of Artificial Sequence:cleavage sequence
<400> 11
   His Pro Phe His Leu Leu Val Tyr
    1               5
<210> 12
   <211> 35
   <212> DNA
   <213> Artificial Sequence
<220>
   <223> Description of Artificial Sequence:primer N1
<400> 12
   aaaaaagcgg ccgcgacaat tcgcgcgcga aggcg 35
<210> 13
   <211> 36
   <212> DNA
   <213> Artificial Sequence
<220>
   <223> Description of Artificial Sequence:primer N2
<400> 13
   aaaaaagcgg ccgctcactg cccgctttcc agtcgg 36